# EUROPEAN PATENT APPLICATION

(11) **EP 2 343 009 A1**
(43) Date of publication of application: **13.07.2011**
(21) Application number: 10016143.9
(22) Date of filing: 28.12.2010
(51) Int. Cl.: A61B 5/022

(54) **Wrist blood pressure monitor**

(30) Priority: 08.01.2010 JP 2010002786
(71) Applicant: Panasonic Electric Works Co., Ltd., Kadoma-shi Osaka (JP)
(72) Inventor: Izumi, Tomohiro, Kadoma-shi (JP); Fumuro, Shinichi, Kadoma-shi (JP); Mizuuchi, Akihiro, Kadoma-shi (JP)
(74) Representative: Samson & Partner

(57) **Abstract**

A wrist blood pressure monitor for use in measuring blood pressure from a wrist of a user includes: a measurement unit for measuring blood pressure; a detection unit for detecting a posture of the user; and a comparison unit for comparing the posture detected by the detection unit with a pre-stored ideal posture to prepare posture information based on the comparison result. Further, the wrist blood pressure monitor includes a notification unit for informing the user of the posture information; and a result storage unit for storing record posture information obtained based on postures detected by the detection unit in association with measurement results of blood pressure measured by the measurement unit.

## Description

### Field of the Invention

The present invention relates to a wrist blood pressure monitor for use in measuring blood pressure from a wrist of a user.

### Background of the Invention

Typical wrist blood pressure monitors include a measurement unit for measuring blood pressure and a display unit capable of displaying the measurement results of blood pressure measured by the measurement unit. As one example of the typical wrist blood pressure monitors, there is known a wrist blood pressure monitor including a detection unit for detecting a posture of a user, a comparison unit for comparing the detected posture with a pre-stored ideal posture and a display unit based on the comparison result for diagrammatically displaying a message to guide the user's posture to the ideal posture (see, e.g., JP2007-54648A). Using diagrams or other symbols, the wrist blood pressure monitor can advise a user of the guidance message to the ideal posture in a visually recognizable manner. The measurement of blood pressure in the ideal posture (within the extent of the ideal posture) assists in enhancing the measurement accuracy of blood pressure.

In this wrist blood pressure monitor that performs the guidance to the ideal posture, the wrist blood pressure monitor is adapted to start the blood pressure measurement after the lapse of a specified time (e.g., 7 seconds) even if the posture detected by the detection unit fails to reach the ideal posture. In this case, there is posed a problem that the blood pressures measured in postures other than the ideal posture (namely, an inaccurate blood pressure) will be checked later.

### Summary of the Invention

In view of the above, the present invention provides a wrist blood pressure monitor capable of analyzing the previously measured blood pressure in relation to the accuracy (reliability) thereof.

In accordance with an aspect of the present invention, there is provided a wrist blood pressure monitor for use in measuring blood pressure from a wrist of a user, including: a measurement unit for measuring blood pressure; a detection unit for detecting a posture of the user; and a comparison unit for comparing the posture detected by the detection unit with a pre-stored ideal posture to prepare posture information based on the comparison result. Further, the wrist blood pressure monitor includes a notification unit for informing the user of the posture information; and a result storage unit for storing record posture information obtained based on postures detected by the detection unit in association with measurement results of blood pressure measured by the measurement unit.

With this configuration, the result storage unit stores the record posture information obtained based on the posture detected by the detection unit, in association with measurement results of blood pressure measured by the measurement unit. Therefore, it is possible to confirm the measurement results of blood pressure together with the record posture information thereof in the future. Since the record posture information is obtained based on the measuring time posture that reflects the accuracy (reliability) of the measurement results of blood pressure, it is possible to analyze the past measurement results of blood pressure in consideration of the accuracy (reliability) thereof.

Further, the wrist blood pressure monitor may include a simultaneous display unit for simultaneously displaying a measurement result of blood pressure and record posture information corresponding thereto.

With this configuration, since the simultaneous display unit simultaneously displays the measurement result of blood pressure and the record posture information corresponding thereto, it is possible to recognize, at a glance, the past measurement results of blood pressure with the accuracy (reliability) thereof.

The wrist blood pressure monitor further may include an operation unit for performing a desired calculation on a set of measurement results of blood pressure based on their record posture information.

With this configuration, since the operation unit performs a calculation on a set of measurement results of blood pressure based on their record posture information, it is possible to perform the calculation by taking into account the accuracy (reliability) of the set of blood pressure measurement results.

The operation unit may calculate an average value for the set of the blood pressure measurement results, the average value for the set being an average value of measurement results of the set except one or more measurement results of the set measured when their postures detected by the detection unit do not match the ideal posture.

With this configuration, the operation unit calculates an average value of the measurement results of the set except the measurement result of the set measured in the state where the posture detected by the detection unit does not match the ideal posture. Therefore, it is possible to increase the accuracy (reliability) of the average value.

The operation unit may calculate average values for a plurality of sets of blood pressure measurement results, and the wrist blood pressure monitor further may include a simultaneous average value display unit for simultaneously displaying average values for a plurality of sets of measurement results.

With this configuration, the operation unit calculates average values for a plurality of sets of blood pressure measurement results and the wrist blood pressure monitor further includes the simultaneous average value display unit for simultaneously displaying the average values. Therefore, it is possible to simultaneously compare and analyze a plurality of average values with increased accuracy (reliability).

Preferably, record posture information for each measurement result of blood pressure is based on the posture detected when the measurement unit starts measuring blood pressure.

With this configuration, since the record posture information is based on only the posture measured when the measurement is started by the measurement unit, it is possible to simplify the control, reduce the quantity of data and eventually realize cost-effectiveness as compared with the case where the posture information is based on all the postures available during the measurement.

Further, record posture information for each measurement result of blood pressure may be based on all the postures detected while the measurement unit measures the blood pressure.

With this configuration, when a posture detected by the detection unit does not temporarily (momentarily) match the ideal posture, it is possible for the result storage unit to store the temporarily mismatching posture as one of the record posture information. This makes it possible to scrutinize the past measurement results of blood pressure in consideration of the accuracy (reliability) thereof.

### Brief Description of the Drawings

The objects and features of the present invention will become apparent from the following description of preferred embodiments, given in conjunction with the accompanying drawings, in which:
Fig. 1 is a schematic view showing a wrist blood pressure monitor worn on the wrist in accordance with an embodiment of the present invention;
Fig. 2 is a block diagram showing the wrist blood pressure monitor of the embodiment;
Figs. 3A and 3B are side and front views showing the main body of the wrist blood pressure monitor in accordance with the embodiment;
Fig. 4 is a front view showing the main body of the wrist blood pressure monitor in accordance with the embodiment;
Fig. 5 is another front view showing the main body of the wrist blood pressure monitor of the embodiment;
Fig. 6 is a further front view showing the main body of the wrist blood pressure monitor of the embodiment; and
Fig. 7 is a still further front view showing the main body of the wrist blood pressure monitor of the embodiment.

### Detailed Description of the Preferred Embodiments

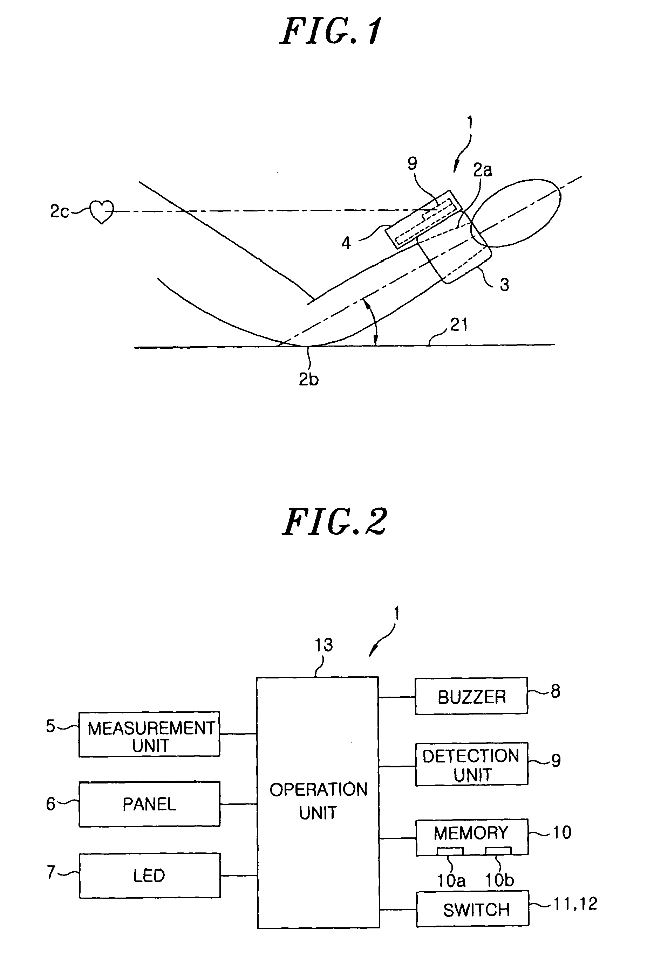

A preferred embodiment of the present invention will now be described with reference to Figs. 1 through 7.

Referring to Fig. 1, the wrist blood pressure monitor (sphygmomanometer) 1 in accordance with an embodiment of the present invention includes a cuff 3 wrapped around the wrist 2a of a user and a main body 4 attached to the cuff 3.

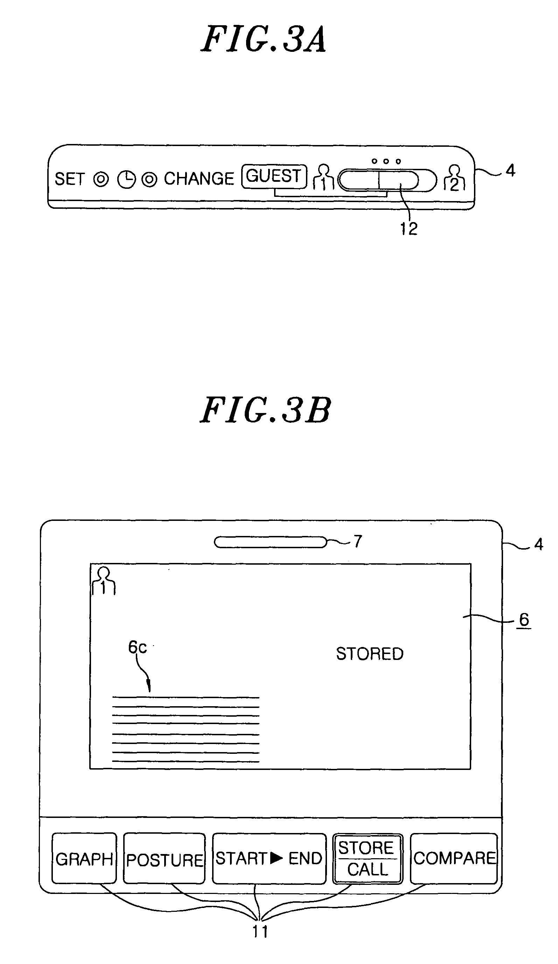

As shown in Figs. 2 and 3, the main body 4 includes a measurement unit 5 for measuring blood pressure, a segment liquid crystal panel 6, a light emitting diode (LED) 7, a buzzer 8, a detection unit 9 for detecting a posture of the user, a memory 10, push button type switches 11, a changeover type switch 12 and an operation unit 13.

The measurement unit 5 includes a pressure adjustment device for performing pressure adjustment through the control of an air filled in the cuff 3 and a sensor for detecting a flow of blood. Using the pressure adjustment device and the sensor, the measurement unit 5 can measure blood pressure. The measurement unit 5 employed in the present embodiment is capable of measuring a pulse rate in company with the blood pressure.

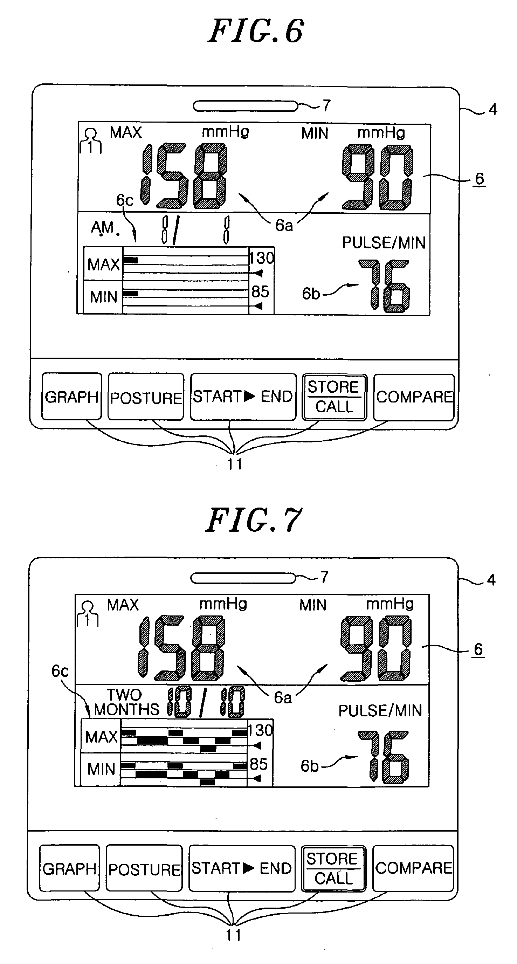

As shown in Fig. 3B, the segment liquid crystal panel 6 is provided on a surface of the main body 4 (namely, a front surface opposite to the attachment surface of the cuff 3 and readily visible to the user). The segment liquid crystal panel 6 includes a seven-segment type blood pressure display unit 6a (see Fig. 6) capable of displaying, in numerals, measurement results of blood pressure measured by the measurement unit 5. The segment liquid crystal panel 6 further includes a seven-segment type pulse rate display unit 6b (see Fig. 6) for displaying measurement results of pulse rate together with the blood pressure.

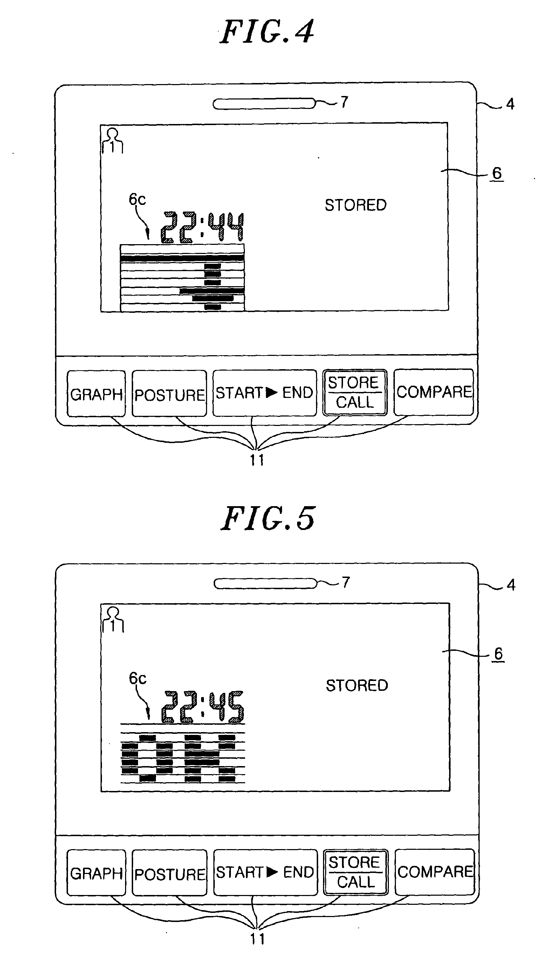

The segment liquid crystal panel 6 further includes a dot display unit 6c (Figs. 4 through 7) having a plurality of dots arranged in rows and columns. The dot display unit 6c has 64 dots in total, i.e., in 8 columns and 8 rows. The dot display unit 6c is capable of graphically representing the measurement results of blood pressure (including desired calculation results thereof) as illustrated in Figs. 6 and 7. In addition, the dot display unit 6c is capable of diagrammatically displaying guide information serving as posture information to be set forth below (see Figs. 4 and 5) .

As shown in Fig. 3B, the light emitting diode 7 is provided above the segment liquid crystal panel 6 on the front surface of the main body 4.

Upon operating the switches 11 or each time the pulse is measured by the measurement unit 5, the buzzer 8 makes an electronic sound like a "beep".

The detection unit 9 uses an acceleration (angle) sensor to detect a posture of the user in the present embodiment. In specific, the height from the surface 21 on which the elbow 2b of the user lies, to the main body 4 (or the wrist 2a) is detected by the detection unit 9 as the user's posture.

The memory 10 stores various data including an ideal posture. In accordance with the present embodiment, the memory 10 includes an ideal posture storage unit for storing, as the ideal posture, the height from the surface 21 to the main body 4 when the main body 4 (or the user's wrist 2a) is raised to the height of the user's heart 2c. The ideal posture storage unit (or the memory 10) further includes a first storage unit 10a for storing a first ideal posture for an unspecific user (hereinafter, referred to a "guest") and a second storage unit 10b for storing a second ideal posture for a specific user.

In the present embodiment, the second storage unit 10b is designed to store the second ideal postures for two specific users. If the changeover type switch 12 (see Fig. 3A) is shifted to the left side, the second storage unit 10b can perform recording (storing) and reading for a first specific user "1". If the changeover type switch 12 is shifted to the right side, the second storage unit 10b can perform recording and reading for a second specific user "2".

The memory 10 also includes a result storage unit for storing record posture information obtained based on the posture detected by the detection unit 9 in association with the measurement results of blood pressure measured by the measurement unit 5, the record posture information being described later.

The operation unit 13 includes a microprocessor and a driver for driving the segment liquid crystal panel 6. The operation unit 13 performs various kinds of calculations and processing.

Next, description will be made on operation of the wrist blood pressure monitor 1 of the present embodiment.

In case where the wrist blood pressure monitor 1 is used by a specific user, e.g., the owner of the wrist blood pressure monitor 1, an ideal posture for the specific user (the second ideal posture) can be stored in the second storage unit 10b. The operation performed at this time will be described.

If the user raises his/her own wrist 2a to the height of the heart 2c and presses a "posture" button of the switch 11 (see Fig. 3B) serving as a manipulation unit (e.g., for two seconds), the operation unit 13 serves as a setting unit and stores a value based on the posture detected by the detection unit 9, e.g., the height of the main body 4, in the second storage unit 10b as the second ideal posture.

In the present embodiment, the second ideal posture has a tolerable range running slightly up and down from the height of the wrist 2a (or the main body 4), i.e., the value based on the posture detected by the detection unit 9. The tolerable range of the second ideal posture is set narrower than that of the first ideal posture. The tolerable range of the first ideal posture is broadly set so that it can correspond to a guest.

If the second ideal posture is stored, the operation unit 13 controls the segment liquid crystal panel 6 to display the word "stored" as illustrated in Fig. 3B. Fig. 3B shows a state that the changeover type switch 12 (see Fig. 3A) is shifted to the left side and the second ideal posture for the first specific user "1" has been written (or stored) .

Next, description will be made on the operation of measuring blood pressure of the user.

If the user wears the wrist blood pressure monitor and operates (or presses) a "start**→**end" button of the switch 11 (see Fig. 3B), the operation unit 13 functions as a comparison unit comparing a posture detected by the detection unit 9 with the pre-stored ideal posture (the first ideal posture or the second ideal posture). Further, the operation unit 13 prepares the posture information based on the comparison result.

In the present embodiment, the posture information includes a determination result on whether the posture detected by the detection unit 9 matches the ideal posture. Further, if the posture detected by the detection unit 9 does not match the ideal posture, the posture information includes guide information for guiding the user's posture to the ideal posture. The user can come close to the ideal posture by moving the wrist 2a (or the main body 4) upwardly or downwardly based on the guide information.

An ideal posture used in comparison is selected from the first ideal posture (namely, the ideal posture for the guest when the changeover type switch 12 is at the middle) and the second ideal postures (namely, the ideal posture for the first specific user "1" available when the changeover type switch 12 is shifted to the left side and the ideal posture for the second specific user "2" available when the switch 12 is shifted to the right side) by operating the changeover type the switch 12.

The operation unit 13 informs the user of the posture information by controlling a notification unit. The notification unit includes the dot display unit 6c of the segment liquid crystal panel 6. The dot display unit 6c displays the posture information.

More specifically, if the posture detected by the detection unit 9 does not match the ideal posture (does not fall within the tolerable range of the ideal posture) and if the detected posture can come closer to the ideal posture though the downward movement of the wrist 2a (or the main body 4), the dot display unit 6c displays a downwardly-facing arrow (diagram) for guiding the user to move down the wrist 2a as illustrated in Fig. 4.

On the contrary, if the detected posture can come closer to the ideal posture though the upward movement of the wrist 2a (or the main body 4), the dot display unit 6c displays an upwardly-facing arrow (diagram) (not shown) for guiding the user to move up the wrist 2a. If the posture detected by the detection unit 9 matches the ideal posture (falls within the tolerable range of the ideal posture), the dot display unit 6c displays a word "OK" as illustrated in Fig. 5.

In the present embodiment, the dot display unit 6c may display the posture information (the guide information) by repeatedly turning on and off activated dots. Alternatively, the dot display unit 6c may display the posture information by flickering of the arrow (diagram) or a moving picture in which an activated dot is shifted in, e.g., the vertical direction.

The notification unit of the present embodiment further includes the light emitting diode (LED) 7 to inform the user of the posture information in addition to the dot display unit 6c. If the posture detected by the detection unit 9 matches the ideal posture (falls within the tolerable range of the ideal posture), the light emitting diode 7 is turned on, for example, thereby informing the user of the posture information. If the posture detected by the detection unit 9 does not match the ideal posture (does not fall within the tolerable range of the ideal posture), the light emitting diode 7, e.g., flickers to inform the user of the posture information.

Meanwhile, if the posture detected by the detection unit 9 matches the ideal posture (falls within the tolerable range of the ideal posture), the operation unit 13 begins to measure blood pressure.

When the posture detected by the detection unit 9 does not match the ideal posture, the operation unit 13 repeatedly performs the processing for guiding the posture of the user such as the notification of the guide information. If the posture detected by the detection unit 9 does not match the ideal posture even after the lapse of a predetermined time period (e.g., 7 seconds), the operation unit 13 begins to measure blood pressure in that state.

When the blood pressure and the pulse rate are measured by the measurement unit 5, the operation unit 13 controls the blood pressure display unit 6a and the pulse rate display unit 6b of the segment liquid crystal panel 6 to display the measurement results of blood pressure and pulse rate, as illustrated in Fig. 6. Further, the operation unit 13 controls the dot display unit 6c of the segment liquid crystal panel 6 to graphically display the measurement results of blood pressure as illustrated in Fig. 6.

The operation unit 13 stores the measurement results of blood pressure and pulse rate together with the date of measurement in reference to the user (a guest, owner 1 and 2) in the memory 10. At this time, the operation unit 13 stores in the result storage unit of the memory 10 the value (the record posture information) based on the posture detected by the detection unit 9 in association with the measurement results of blood pressure measured by the measurement unit 5. In the present embodiment, a determination result (i.e., a portion of the posture information) on whether the posture detected by the detection unit 9 matches the ideal posture is stored as the record posture information, which is based on the posture of the user measured when (or just before) the measurement unit 5 starts measuring the blood pressure.

The measurement results of blood pressure measured as above can be displayed later in many different patterns.

For example, one of the past measurement results of blood pressure can be selected and displayed by operating the switches 11. In the wrist blood pressure monitor 1 of the present embodiment, measurement results of blood pressure and their record posture information (i.e., determination results on whether the postures detected by the detection unit 9 match the ideal posture) are simultaneously displayed by both the segment liquid crystal panel 6 and the light emitting diode 7.

To that end, there is provided a simultaneous display unit including the segment liquid crystal panel 6 and the light emitting diode 7. Specifically, if the posture detected by the detection unit 9 at the measurement startup time matches the ideal posture, the operation unit 13 turns on the light emitting diode 7 to simultaneously deliver such message (the record posture information) to the user when the measurement result of blood pressure is displayed on the blood pressure display unit 6a of the segment liquid crystal panel 6. If the posture detected by the detection unit 9 at the measurement startup time does not match the ideal posture, the operation unit 13 flickers the light emitting diode 7 to simultaneously deliver such message (the record posture information) to the user when the measurement result of blood pressure is displayed on the blood pressure display unit 6a of the segment liquid crystal panel 6.

By selectively operating the switches 11, it is possible to have the operation unit 13 functioning as a calculation unit perform a desired calculation (operation) on the past measurement results of blood pressure and to display the calculation result. At this time, the operation unit 13 performs the calculation on the blood pressure measurement results based on their record posture information.

More specifically, the operation unit 13 may calculate an average value for the measurement results of blood pressure except measurement results measured in the state where the posture detected by the detection unit 9 does not match the ideal posture. Alternatively, the operation unit 13 may calculate average values for a plurality of sets of the blood pressure measurement results. In this case, the average values for a plurality of the sets may be simultaneously displayed in the dot display unit 6c functioning as a simultaneous average value display unit.

For example, the operation unit 13 may calculate weekly average values for the blood pressure measurement results (except the blood pressure measurement result measured in the state where the posture detected by the detection unit 9 does not match the ideal posture) for eight weeks. As shown in Fig. 7, all of the weekly average values thus calculated may be graphically displayed in the dot display unit 6c from the left side toward the right side. Other examples of average values for a plurality of sets include average values calculated on a time zone basis, e.g., forenoon average values.

Next, description will be made on the advantageous effects achieved by the above-mentioned embodiments.
(1) Since the memory 10 (the result storage unit) stores the posture information obtained based on the posture detected by the detection unit 9 in association with the measurement results of blood pressure measured by the measurement unit 5, it is possible to confirm the measurement results of blood pressure together with their record posture information later. Because the record posture information is based on the measuring time posture that reflects the accuracy (reliability) of the measurement results of blood pressure, it is possible to analyze the past measurement results of blood pressure in consideration of the accuracy (reliability) thereof.
(2) Since there is provided the simultaneous display unit including the segment liquid crystal panel 6 and the light emitting diode 7 to simultaneously display the record posture information and the measurement results of blood pressure, it is possible to recognize, at a glance, the past measurement results of blood pressure together with the accuracy (reliability) thereof.
(3) Since the operation unit 13 can perform a desired calculation on the measurement results of blood pressure based on their record posture information, it is possible to perform the calculation by taking into account the accuracy (reliability) of the measurement results of blood pressure. More specifically, the operation unit 13 calculates an average value of the measurement results of blood pressure except the measurement result of blood pressure measured in the state where the posture detected by the detection unit 9 does not match the ideal posture. This makes it possible to increase the accuracy (reliability) of the average value.
(4) The operation unit 13 is capable of calculating average values for a plurality of sets of the blood pressure measurement results. The wrist blood pressure monitor 1 includes the dot display unit 6c (the simultaneous average value display unit) for simultaneously displaying the average values for a plurality of the sets. Accordingly, it is possible to readily compare and analyze the average values with increased accuracy (reliability) thereof.
(5) The record posture information stored in the memory 10 (the result storage unit) in association with the measurement results of blood pressure measured by the measurement unit 5 is based on the posture detected when the measurement unit 5 starts to measure the blood pressure. Therefore, as compared with the case where the posture information is based on all the postures available during the measurement, it is possible to simplify the control, reduce the quantity of data and eventually realize cost-effectiveness.

The embodiment described above may be modified as follows.

While the wrist blood pressure monitor 1 of the foregoing embodiment has the simultaneous display unit including the segment liquid crystal panel 6 and the light emitting diode 7 to simultaneously display the record posture information and the measurement results of blood pressure, the present invention is not limited thereto. Alternatively, the wrist blood pressure monitor 1 may display either of the record posture information and the blood pressure measurement results. The simultaneous display may be performed using other units and methods (for example, both of the record posture information and the blood pressure measurement results may be displayed in the segment liquid crystal panel 6).

While the operation unit 13 of the foregoing embodiment performs a desired calculation on the measurement results of blood pressure based on the record posture information, the present invention is not limited thereto. The desired calculation may be performed regardless of the record posture information. More specifically, the average value of the blood pressure measurement results may be calculated without excluding the measurement result of blood pressure measured in the state where the posture detected by the detection unit 9 does not match the ideal posture. Needless to say, such calculations may be selectively performed at the user's choice.

While the operation unit 13 is adapted to calculate average values for a plurality of sets of the blood pressure measurement results and the dot display unit 6c (the simultaneous average value display unit) is provided to simultaneously (and graphically) display the average values, the present invention is not limited thereto. Alternatively, the simultaneous display of average values may be performed by a plurality of seven-segment type display units.

While the record posture information stored in the memory 10 (the result storage unit) in association with the measurement results of blood pressure measured by the measurement unit 5 is based on the posture detected at the measurement startup time, the present invention is not limited thereto. For example, the record posture information may be based on all the postures detected while the measurement made by the measurement unit 5. Accordingly, when a posture detected by the detection unit 9 does not temporarily (momentarily) match the ideal posture, it is possible for the memory 10 (the result storage unit) to store the temporarily mismatching posture as one of the record posture information. This makes it possible to scrutinize the past measurement results of blood pressure in consideration of the accuracy (reliability) thereof.

While only the determination result on whether a posture detected by the detection unit 9 does not match the ideal posture is stored as the record posture information in the memory 10 (the result storage unit), the difference value (i.e., the degree of deviation) between the posture detected by the detection unit 9 and the ideal posture may be stored as the record posture information.

Although the segment liquid crystal panel 6 is used in the foregoing embodiment, it is possible to use, e.g., a dot matrix type liquid crystal panel or other display units.

While the memory 10 (the ideal posture storage unit) of the foregoing embodiment includes the first storage unit 10a for storing a first ideal posture corresponding to an unspecified guest and the second storage unit 10b for storing a second ideal posture corresponding to a specific user, the present invention is not limited thereto. For example, the memory 10 may not be provided with the second storage unit 10b.

In the foregoing embodiment, a posture detected by the detection unit 9 is stored in the second storage unit 10b as the second ideal posture by operating the switch 11 (the manipulation unit). However, the present invention is not limited thereto. The second ideal posture may be manually inputted.

In the foregoing embodiment, the height from the surface 21 on which the elbow 2b of the user lies, to the main body 4 (or the wrist 2a) is detected as a user's posture and is used in performing the respective processing. However, the present invention is not limited thereto, values based on other postures of the user may be detected as the user's posture and used in performing the respective processing. Further, other values may be used in combination with the height of the wrist 2a. In this case, the ideal posture data stored in the memory 10 need to be changed into values detected based on the ideal postures corresponding to other postures and the detection unit 9 needs to be made capable of detecting other postures.

While the invention has been shown and described with respect to the embodiments, various changes and modification may be made without being limited thereto. Further, the embodiment of the present invention is not limited to the above-described embodiment.

## Claims

1. A wrist blood pressure monitor for use in measuring blood pressure from a wrist of a user, comprising:
a measurement unit for measuring blood pressure;
a detection unit for detecting a posture of the user;
a comparison unit for comparing the posture detected by the detection unit with a pre-stored ideal posture to prepare posture information based on the comparison result;
a notification unit for informing the user of the posture information; and
a result storage unit for storing record posture information obtained based on postures detected by the detection unit in association with measurement results of blood pressure measured by the measurement unit.

2. The wrist blood pressure monitor of claim 1, further comprising a simultaneous display unit for simultaneously displaying a measurement result of blood pressure and record posture information corresponding thereto.

3. The wrist blood pressure monitor of claim 1 or 2, further comprising an operation unit for performing a desired calculation on a set of measurement results of blood pressure based on their record posture information.

4. The wrist blood pressure monitor of claim 3, wherein the operation unit is adapted to calculate an average value for the set of the measurement results, the average value for the set being an average value of measurement results of the set except one or more measurement results of the set measured when their postures detected by the detection unit do not match the ideal posture.

5. The wrist blood pressure monitor of claim 4, wherein the operation unit is adapted to calculate average values for a plurality of sets of blood pressure measurement results, and
the wrist blood pressure monitor further comprises a simultaneous average value display unit for simultaneously displaying the average values for the sets.

6. The wrist blood pressure monitor of any one of claims 1 to 5, wherein record posture information for each measurement result of blood pressure is based on the posture detected when the measurement unit starts measuring the blood pressure.

7. The wrist blood pressure monitor of any one of claims 1 to 5, wherein record posture information for each measurement result of blood pressure is based on all the postures detected while the measurement unit measures the blood pressure.
